# EUROPEAN PATENT SPECIFICATION

(11) **EP 0 654 480 B1**
(45) Date of publication and mention of the grant of the patent: **27.02.2002**
(21) Application number: 94116421.2
(22) Date of filing: 18.10.1994
(51) Int. Cl.: C07K 5/06, C07K 1/00

(54) **Process for producing alpha-L-aspartyl-L-pheylalanine methyl ester**
Verfahren zur Herstellung von alpha-L-Aspartyl-L-Phenylalanin Methyl Ester
Procédé pour la préparation de alpha-L-aspartyl-L-phenylalanine methyl ester

(30) Priority: 19.11.1993 JP 29045293
(43) Date of publication of application: 24.05.1995
(73) Proprietor: Ajinomoto Co., Inc., Tokyo 104 (JP)
(72) Inventor: Kataoka, Takehiko, c/o Central Research Lab., Kawasaki-shi, Kanagawa-ken (JP); Kishimoto, Shinichi, c/o Central Research Lab., Kawasaki-shi, Kanagawa-ken (JP); Sato, Osahiro, c/o Central Research Lab., Kawasaki-shi, Kanagawa-ken (JP)
(74) Representative: Strehl Schübel-Hopf & Partner

(56) References cited:
- EP-A- 0 514 936

## Description

### Field of the Invention

This invention relates to a process for producing α-L-aspartyl-L-phenylalanine methyl ester (hereinafter referred to as α-APM), more particularly, to a significantly improved process for producing α-APM by solid-liquid separation of the aqueous suspension thereof.

α-APM obtained according to the present invention is expected to be widely utilized as a low caloric dietary sweetener which is about 200 times as sweet as sugar.

### Description of Prior Art

For industrial production of α-APM, following processes are known:
(1) a process which comprises coupling N-protected L-aspartic anhydride with L-phenylalanine methyl ester in an organic solvent, followed by deprotection (USP No. 3786039),
(2) a process wherein α-L-aspartyl-L-phenylalanine is converted to methyl ester thereof in a mixed solvent consisting of water, methanol and hydrochloric acid to obtain α-APM hydrochloride, which is neutralized to obtain α-APM (Japanese Patent Application Laid-open No. 82752-1978), and
(3) a process which comprises condensing N-protected L-aspartic anhydride with phenylalanine methyl ester in the presence of an enzyme, followed by deprotection (Japanese Patent Publication No. 135595-1980).

Any of these processes are generally carried out with a crystallization step for purification. For example, in the chemical synthesis such as the above process (1), as for means to selectively remove impurities including β-isomer (β-L-aspartyl-L-phenylalanine methyl ester), which is an inevitably obtained by-product, a process is known wherein α-APM containing impurities is contacted with hydrohalogenic acid to crystallize the product as a hydrohalide salt. Hydrohalide salts of α-APM obtained in the form of wet crystals are subsequently dissolved or suspended in an aqueous solvent which is neutralized by adding a base to separate α-APM as wet crystals. For further purification, the crystals of α-APM obtained may sometimes be redissolved and recrystallized. These operations may be carried out by a continuous process or a batch process. An optional recrystallization step may be carried out. The final product is usually a dried product. Such dried product is generally obtained by drying the wet crystals after the last crystallization.

The aforementioned various operations for crystallization may be generally conducted in a crystallization tank accompanied with forced flow such as stirring or external circulation. When α-APM is crystallized under forced flow, it is known that solid-liquid separation and dehydration properties of the resulting suspension are extremely poor. For example, in an industrial scale, when the suspension (1.3 m³) obtained by cooling a 3.5 wt% solution of α-APM from 65 to 5°C is separated using a centrifugal filter (diameter, 1,220 mm; height, 500 mm), it requires 2.5 hours and 2 hours for charging of the suspension and dehydration, respectively. Further, the water content of the resulting wet crystals is as high as 55 - 60 % (% used herein means that on wet basis) (Cf. Comparative Example 1).

Longer separation time requires more separators for treatment of a specified amount of suspension, resulting in too large equipment. Further, when the wet crystals after separation are dried, the high water content results in large heat load during the drying step.

In addition to such problems the thus obtained wet crystals with a high water content are very sticky and show problems, for example, due to adhesion of crystals to the wall of the apparatus during the transporting and drying steps. Such problems become severe particularly when the wet crystals after separation are continuously dried. That is, the operation to remove crystals adhering to the continuous transport line, feeder to the dryer, and internal surface of the dryer is required at high frequency. Too much adhesion makes the continuous operation impossible. The water content of the wet crystals of around 40 % or more is considered to cause problems. As mentioned above, it is extremely difficult to reduce the water content of the wet crystals to not more than 50 % so far as separation is carried out using a centrifugal filter.

Such problems may occur, more or less, not only when using a centrifugal filter but also in the case where a filter separator that performs reduced pressure filtration such as a continuous belt filter or Oliver filter is used.

### Problem to be solved by the Invention

The problems to be solved by the invention are as follows:
(1) Separation time for solid-liquid separation of α-APM is long;
(2) Heat load for drying is great due to high water content of the wet crystals obtained by separation; and
(3) Adhesion of the crystals to the internal surface of apparatus becomes significant during transportation and drying because the wet crystals obtained by separation are very sticky.

### Means to solve the Problem

The present inventors have studied intensively to solve the aforementioned problems in solid-liquid separation of an aqueous suspension of α-APM. As the result, they have surprisingly found that wet crystals having a water content of not more than 40% can be obtained by compression of the suspension under high pressure of not less than 20 kg/cm² (20x10⁵ Pa). Generally the dehydration degree of the separated wet crystals becomes constant when the pressure for compression increases to about 15 kg/cm² (15x10⁵ Pa). However, in the case of α-APM, since the compressibility of the wet crystal cake is extremely high, the compression shows a significant effect under high pressure of not less than 20 kg/cm². The thus obtained wet crystals are less sticky, hence a continuous drying step can be conducted over a long period of time without any problems.

In addition, when the compression pressure is set extremely high, i.e. not less than 50 kg/cm² (5x10⁶ Pa), the water content of the wet crystals is reduced to 30 % or lower and the heat load during drying has been found to be significantly reduced.

The time necessary for such compression and separation is about 5 min. even when the thickness of the wet crystal layer is about 5 - 6 mm. Thus, the filtration area required for treatment of a specified amount of the suspension can be also reduced.

In addition, it is found that compression is expected to be effective for dehydration and removal of stickiness of the wet crystals, even when the aqueous suspension of α-APM obtained by crystallization is once subjected to solid-liquid separation by a method other than compression separation, and the obtained wet crystals are compressed directly or after mixing with water by kneading, resuspending or the like.

The present inventors have solved the above problems by applying these novel findings to the practical process for production of α-APM, rationalized the steps, and attained the present invention.

That is, the present invention is directed to a process for producing α-APM by solid-liquid separation of an aqueous suspension of α-APM, wherein said aqueous suspension of wet crystals is pressed so that the final pressure for compression is not less than 20 kg/cm² (20x10⁵ Pa) and the wet crystals after pressing are dried.

The process for compression according to the present invention is not particularly limited so far as the final pressure for compression is not less than 20 kg/cm². For example, a process wherein said suspension is supplied into the gap of a double cylinder of which the inside or outside cylinder is equipped with filter cloth, and pressure is applied from the cylinder without filter cloth, or a process wherein said suspension is supplied into a cylinder covered with filter cloth on one side and pressure is applied from the other side using a piston may be employed. The pressure may be not less than 20 kg/cm² from the beginning of separation, or separation is started at low pressure and after cake is formed to some extent, the pressure may be increased to 20 kg/cm² or more. Considering the possibility of leakage of the suspended crystals into filtrate during compression, the filter cloth used is desired to have an air permeability of not more than 3 cc/cm²·sec., and is preferably a plain or twilled weave.

As mentioned above, the compression may be conducted after an aqueous suspension of α-APM has been supplied directly to a compression device. Alternatively, said aqueous suspension is once separated by means other than compression and the resulting wet crystals are supplied to the compression device, then the compression is conducted. For the purpose of, for example, increasing the flowability of the feed crystal, enhancing washing efficiency of the wet crystals or the like, said wet crystal can be supplied after kneading with water, or supplied after resuspending in water in the latter process. As means for separation other than compression, for example, continuous filtration under reduced pressure can be employed.

The thickness of the wet crystal layer during compression is not particularly limited. Considering the effect of compression and for easy removal of the wet crystal layer from the filter cloth, the thickness is desirably 4 mm or more at a pressure of 20 kg/cm² (20x10⁵ Pa), and 3 mm or more at a pressure of 50 kg/cm² (50x10⁵ Pa). The concentration of the suspension supplied to the compression device is not particularly limited so long as wet crystals of such thickness can be obtained. Even a suspension with a low concentration can be employed by pre-filtration of the suspension in the device before the compression step by applying liquid pressure of several kilograms per cm² to the suspension when it is supplied to the device.

In some cases, the wet crystals are required to be washed during compression operation. This can be carried out by substitution washing operation wherein a wet crystal cake is firstly formed at a relatively low pressure of 10 - 50 kg/cm² ((10 - 50) x 10⁵ Pa) then the separator is filled with a washing liquid, subsequently the washing liquid is passed through the wet crystal cake at relatively high pressure of 50 - 100 kg/cm².

When the wet crystals after compression are dried, they are desirably dried continuously to minimize the drier size considering lowering costs for construction. The wet crystals obtained according to the present invention are almost free from the problems of adhesion to the device, therefore such continuous drying process can be readily conducted. The temperature of the hot air is desirably not lower than 50 °C considering drying efficiency. The dried product can be obtained as powder by a process involving a grinding step before or after the drying step. Alternatively, a method involving a granulation step results in a product in the form of granules.

### Advantages of the Invention

In the industrial process for production of α-APM by solid-liquid separation of an aqueous suspension of α-APM, wet crystals with low water content and free from adhesion can be obtained according to the present invention. Accordingly, the heat load during drying of the wet crystals can be considerably reduced, and drying can be conducted continuously. Further, the equipment required is reduced because of reduced time required for separation. Therefore, the present process is practically valuable because steps are greatly rationalized.

### Examples

The present invention will be explained in detail in the following examples.

The suspension used in the following examples (hereinafter referred to as 'suspension 1') was prepared as follows. That is, 3.5 wt% solution of α-APM (19 m³, liquid temperature, 65 °C) is charged in a 21 m³ stirring tank having cooling surface, and cooled from 65°C to 5 °C over a period of 6 hours with stirring for crystallization.

### Example 1

Suspension 1 was filtered under reduced pressure to prepare a high concentration suspension (three-fold concentrate, hereinafter referred to as 'suspension 2'). Suspension 2 (180 g) was supplied in a piston type press filter (cylinder diameter, 75 mm) wherein filtrate was extruded with a piston from one side to the opposite side of the cylinder equipped with a filter cloth. After compressing for 3 minutes at varying pressure, the water content of the wet crystals was measured according to Karl Fischer's method. The results are shown in Table 1.

**Table 1**

| | | | | |
|---|---|---|---|---|
| Pressure [Kg/cm² | 10 | 20 | 50 | 100 |
| Thickness of Wet Crystals [mm] | 5.5 | 4.0 | 3.5 | 3.0 |
| Water Content [%] | 55 | 38 | 29 | 23 |

### Example 2

Suspension 1 was separated using a Tube Press type TPM manufactured by Asizawa (filtration area, 0.9 m², charged amount, 35 ℓ), which was an industrial compression separator. This separator, a double cylinder type device, was equipped with a filter cloth in the inside cylinder and diaphragm for compression in the outside cylinder. The suspension supplied between them was compressed from the outside cylinder.

Firstly, the suspension (180 ℓ) was supplied in the separator at the pressure of 5 kg/cm². The charged volume of the device was 35 ℓ. That is, about 5-time pre-concentration was completed in the separator while supplying. It took 5 minutes for supplying.

The supplied suspension was compressed and separated at the pressure of 50 kg/cm² for 5 minutes. Subsequently, the inside cylinder was slid downwardly while wet crystals adhered to the filter cloth. Then air was blown from the inside to peel off the wet crystals. The peeling performance was good. Wet crystals of 5 - 6 mm thickness with average water content of 28 % were obtained. Time required for peeling was 3 minutes.

Subsequently, separation operations were repeated for 12 hours in the same manner. As the result, 8.6 m³ of suspension in total could be treated. It corresponds to 19.1 m³/d·m² calculated as a treated amount per unit filtration area per day.

140 kg (wet basis) of thus obtained wet crystals were continuously dried using a Micron Drier type MDV-1 manufactured by Hosokawa Micron. The hot air inlet temperature was set at 170 - 180 °C and the drying process was conducted for 4 hours. During the process, adhesion of crystals did not cause problems in charge hopper, screw-type feeder, nor drier body. Finally, 95 kg of dried crystals with water content of 2.3 - 2.5 % were obtained. Heat load per 1 kg of the dried crystal was calculated as 238 kcal/kg including heat loss.

### Comparative Example 1

Suspension 1 was separated using a bottom discharging type centrifugal filter (basket diameter, 1,220 mm; basket height, 500 mm; filtration area, 1.9 m²). It took as long as 150 minutes to supply suspension because the liquid was not filtered well. After supply was completed, separation and dehydration operations were conducted for 120 minutes. Water content of the resulting wet crystals was as high as 55 %.

Subsequently, the same separation operation was repeated and three cycles of separation were conducted. The remaining crystal layer which adhered in the clearance between scraper and filter cloth, could not be scraped off in the first cycle and was consolidated thereafter. Accordingly, after the second cycle was completed, the remaining layer must be removed by hand. As the result, it took 15 hours to complete three cycles in total. The total amount of the treated suspension was 3.7 m³, which is calculated to 3.1 m³/d·m² as a treated amount per unit filtration area per day. Water content of the resulting wet crystals was 55 - 60%.

Subsequently, 100 kg (wet basis) of thus obtained wet crystals were dried by a Micron drier type MDV-1 in the same manner as in Example 2. The inlet temperature of the hot air was set at 170 180 °C as in Example 2. The wet crystals were sticky and considerably adhered to the charge hopper and screw-type feeder, making automatic supplying by these devices impossible. Thus, drying step was conducted by extruding the wet crystals into the dryer by manual operation. It required four hours to complete drying steps, affording 33 kg of dried crystal with water content of 2 - 3 %. Average heat load per 1 kg of the dried crystal was calculated to 800 kcal/kg including heat loss.

The results of Example 2 as well as Comparative Example 1 are shown in Table 2.

**Table 2**

| | Ex. 2 | Com.Ex. 1 |
|---|---|---|
| Treated Suspension per unit filtration area [m³/d·m²] | 19.1 | 3.1 |
| Water Content of Wet Crystal [%] | 28 | 55 - 60 |
| Adhesion of Crystal during drying step | small | significant |
| Heat load during drying step [kcal/kg] | 238 | 800 |

## Claims

1. A process for producing dried crystals of α-L-aspartyl-L-phenylalanine methyl ester by solid-liquid separation of an aqueous suspension of α-L-aspartyl-L-phenylalanine methyl ester to obtain wet crystals and drying said wet crystals, wherein said aqueous suspension is subjected to a compression step in which the final compression pressure is not less than 2 x 10⁶ Pa (20 kg/cm²).

2. A process according to claim 1, wherein the final compression pressure in the compression step is not less than 5 x 10⁶ Pa (50 kg/cm²).

3. A process according to claim 1 or claim 2, wherein the wet crystals after the compression step are continuously dried with hot air at a temperature of not less than 50 °C.

4. A process according to any of the claims 1 to 3, wherein said aqueous suspension of α-L-aspartyl-L-phenylalanine methyl ester is subjected to a separation step by filtration before the compression step is carried out.

5. A process according to any of the claims 1 to 4, wherein the crystal suspension subjected to the compression step has been obtained by continuously filtering an aqueous suspension of α-L-aspartyl-L-phenylalanine methyl ester under reduced pressure.

6. A process according to any of the claims 1 to 5, wherein said aqueous suspension of α-L-aspartyl-L-phenylalanine methyl ester has been obtained by suspending crystals of α-L-aspartyl-L-phenylalanine methyl ester in an aqueous medium.

7. A process according to any of the claims 1 to 5, wherein said aqueous suspension of α-L-aspartyl-L-phenylalanine methyl ester has been obtained from the process of chemical synthesis of α-L-aspartyl-L-phenylalanine methyl ester.

8. A process according to any of the claims 1 to 5, wherein said aqueous suspension of α-L-aspartyl-L-phenylalanine methyl ester has been obtained by a recrystallisation process.

## Patentansprüche

1. Verfahren zur Herstellung von getrockneten Kristallen von α-L-Aspartyl-L-phenylalaninmethylester durch Fest-Flüssig-Trennung einer wässerigen Suspension von α-L-Aspartyl-L-phenylalaninmethylester, wobei feuchte Kristalle erhalten werden, und Trocknen dieser feuchten Kristalle, wobei die wässerige Suspension einer Kompressionsstufe unterworfen wird, in welcher der endgültige Kompressionsdruck nicht weniger als 2 x 10⁶ Pa (20 kg/cm²) beträgt.

2. Verfahren nach Anspruch 1, wobei der endgültige Kompressionsdruck in der Kompressionsstufe nicht weniger als 5 x 10⁶ Pa (50 kg/cm²) beträgt.

3. Verfahren nach Anspruch 1 oder Anspruch 2, wobei die feuchten Kristalle nach der Kompressionsstufe kontinuierlich mit Heißluft bei einer Temperatur von nicht weniger als 50°C getrocknet werden.

4. Verfahren nach einem der Ansprüche 1 bis 3, wobei die wässerige Suspension von α-L-Aspartyl-L-phenylalaninmethylester einer Trennstufe durch Filtration unterworfen wird, bevor die Kompressionsstufe durchgeführt wird.

5. Verfahren nach einem der Ansprüche 1 bis 4, wobei die der Kompressionsstufe unterworfene Kristallsuspension erhalten wurde, indem eine wässerige Suspension von α-L-Aspartyl-L-phenylalaninmethylester unter reduziertem Druck kontinuierlich filtriert wurde.

6. Verfahren nach einem der Ansprüche 1 bis 5, wobei die wässerige Suspension von α-L-Aspartyl-L-phenylalaninmethylester durch Suspendieren von Kristallen von α-L-Aspartyl-L-phenylalaninmethylester in einem wässerigen Medium erhalten wurde.

7. Verfahren nach einem der Ansprüche 1 bis 5, wobei die wässerige Suspension von α-L-Aspartyl-L-phenylalaninmethylester in dem Verfahren der chemischen Synthese von α-L-Aspartyl-L-phenylalaninmethylester erhalten wurde.

8. Verfahren nach einem der Ansprüche 1 bis 5, wobei die wässerige Suspension von α-L-Aspartyl-L-phenylalaninmethylester mit Hilfe eines Umkristallisationsverfahrens erhalten wurde.

## Revendications

1. Procédé pour la production de cristaux séchés d'ester de méthyle de l'α-L-aspartyl-L-phénylalanine par séparation solide-liquide d'une suspension aqueuse d'ester de méthyle de l'α-L-aspartyl-L-phénylalanine pour obtenir des cristaux humides et séchage desdits cristaux humides, dans lequel ladite suspension aqueuse est soumise à une étape de compression dans laquelle la pression finale de compression n'est pas inférieure à 2 x 10⁶ Pa (20 kg/cm²).

2. Procédé selon la revendication 1, dans lequel la pression finale de compression dans l'étape de compression n'est pas inférieure à 5 x 10⁶ Pa (50 kg/cm²).

3. Procédé selon la revendication 1 ou la revendication 2, dans lequel les cristaux humides après l'étape de la compression sont séchés en continu avec de l'air chaud à une température non inférieure à 50°C.

4. Procédé selon l'une quelconque des revendications 1 à 3, dans lequel ladite suspension aqueuse d'ester de méthyle de l'α-L-aspartyl-L-phénylalanine est soumise à une étape de séparation par filtration avant la mise en oeuvre de l'étape de compression.

5. Procédé selon l'une quelconque des revendications 1 à 4, dans lequel la suspension de cristaux soumise à l'étape de compression a été obtenue en filtrant en continu une suspension aqueuse d'ester de méthyle de l'α-L-aspartyl-L-phénylalanine sous pression réduite.

6. Procédé selon l'une quelconque des revendications 1 à 5, dans lequel ladite suspension aqueuse d'ester de méthyle de l'α-L-aspartyl-L-phénylalanine a été obtenue en mettant en suspension des cristaux d'ester de méthyle de l'α-L-aspartyl-L-phénylalanine dans un milieu aqueux.

7. Procédé selon l'une quelconque des revendications 1 à 5, dans lequel ladite suspension aqueuse d'ester de méthyle de l'α-L-aspartyl-L-phénylalanine a été obtenue par le procédé de synthèse chimique de l'ester de méthyle de l'α-L-aspartyl-L-phénylalanine.

8. Procédé selon l'une quelconque des revendications 1 à 5, dans lequel ladite suspension aqueuse d'ester de méthyle de l'α-L-aspartyl-L-phénylalanine a été obtenue par un procédé de recristallisation.
